# FASCICULE DE BREVET EUROPEEN

(11) **EP 3 003 196 B1**
(45) Date de publication et mention de la délivrance du brevet: **17.08.2022**
(21) Numéro de dépôt: 14731720.0
(22) Date de dépôt: 26.05.2014
(51) Int. Cl.: A61F 2/00

(54) **ENSEMBLE MEDICAL COMPRENANT DEUX EMBALLAGES**
MEDIZINISCHER ANORDNUNG MIT ZWEI VERPACKUNGSELEMENTEN
ASSEMBLY INCLUDING TWO PACKAGING ELEMENTS

(30) Priorité: 27.05.2013 FR 1354751
(43) Date de publication de la demande: 13.04.2016
(73) Titulaire: Selenium Medical, 17000 La Rochelle (FR)
(72) Inventeur: RICHART, Olivier, 17580 Le Bois Plage en Ré (FR)
(74) Mandataire: Ipsilon
(86) Numéro de dépôt international: PCT/FR2014/051223
(87) Numéro de publication internationale: WO 2014/191668

(56) Documents cités:
- WO-A1-2010/004330
- WO-A2-2006/116230
- WO-A2-2011/109630
- FR-A1- 2 876 086
- US-A- 5 193 679
- US-A1- 2012 158 128

## Description

La présente invention concerne de manière générale les emballages pour objets médicaux, en particulier pour pièces médicales de préférence stérilisées. On connaît de l'état de la technique un ensemble de double emballage pour objets médicaux qui comprend un premier emballage, dit emballage extérieur, et un deuxième emballage dit emballage intérieur, qui est logé dans l'emballage extérieur et qui contient une pluralité d'objets médicaux. Ledit emballage intérieur se présente sous la forme d'une plaquette dont une face, appelée face d'accès délimite une pluralité de cavités borgnes distinctes qui logent chacune un objet médical. Ladite face d'accès est recouverte d'une feuille de scellage qui vient refermer chaque cavité.

L'emballage extérieur se présente généralement sous forme d'une barquette qui délimite une chambre dans laquelle est logé l'emballage intérieur et qui est refermée par une feuille de scellage. L'utilisation d'un double emballage permet de limiter les risques de contamination des objets médicaux ainsi conditionnés. Cependant, on constate que les objets ainsi conditionnés dans une plaquette scellée et logée dans une barquette elle aussi scellée sont difficilement visibles, de sorte que les utilisateurs perdent un temps précieux pour identifier les objets médicaux dont ils ont besoin.

Le document US5193679A propose un emballage pour prothèse de hanche qui comprend deux parties rabattables l'une sur l'autre, ainsi qu'un manchon distal qui recouvre une partie de la prothèse de hanche qui s'étend en saillie des deux parties d'emballage.

Un emballage selon le préambule de la revendication 1 es connu du document WO-A-2010/004330.

La présente invention a pour but de proposer un nouvel ensemble médical d'emballages contenant une pluralité d'objets médicaux dont la visibilité est améliorée tout en conservant un conditionnement limitant le risque de contamination desdits objets.

A cet effet, l'invention a pour objet un ensemble médical conforme à la revendication 1.

Grâce au pliage des première et deuxième parties de l'emballage intérieur l'une contre l'autre en amenant les parties correspondantes de la feuille de scellage en vis-à-vis, la visibilité du ou des objets contenus dans chaque partie d'emballage est améliorée, puisque ceux-ci sont visibles par transparence des parties d'emballage à travers l'emballage extérieur. La feuille de scellage au moins partiellement opacifiante ne gêne pas la visibilité des objets contenus dans l'emballage intérieur puisque la feuille de scellage se retrouve, par pliage, au centre de l'emballage intérieur plié, et que lesdits objets peuvent être observés à travers les parties transparentes des emballages intérieur et extérieur qui se situent en regard les unes des autres. En outre, le conditionnement sous forme de double emballage et d'un scellage de chaque cavité, permet de limiter le risque de contamination desdits objets.

Selon une caractéristique avantageuse de l'invention, l'emballage intérieur comprend des moyens de saisie accessibles à l'ouverture de l'emballage extérieur.

Selon une caractéristique avantageuse de l'invention, ledit emballage intérieur comprend une troisième partie d'emballage transparente qui s'étend entre les première et deuxième parties dudit emballage intérieur, ladite troisième partie d'emballage comprenant au moins une cavité de réception d'objet qui est refermée par une troisième partie de la feuille de scellage.

Selon une caractéristique avantageuse de l'invention, les première et deuxième parties d'emballage de l'emballage intérieur sont séparées l'une de l'autre par au moins une ligne de pliage.

Selon une caractéristique avantageuse de l'invention, les première et deuxième parties d'emballage sont séparées chacune de la troisième partie d'emballage par deux lignes de pliage parallèles et écartées l'une de l'autre.

Selon une caractéristique avantageuse de l'invention, lesdites parties d'emballage de l'emballage intérieur sont réalisées d'un seul tenant. Préférentiellement l'emballage intérieur se présente sous la forme d'une plaque pliable dans laquelle sont formées lesdites cavités.

Selon une caractéristique avantageuse de l'invention, lesdites parties d'emballage de l'emballage intérieur sont disjointes les unes des autres, et ladite feuille de scellage qui ferme les cavités desdites parties d'emballage relient entre elles lesdites parties d'emballage de l'emballage intérieur.

Préférentiellement, la feuille de scellage de l'emballage intérieur est appliquée sur lesdites parties d'emballage de l'emballage intérieur sous pression et à chaud.

Selon une caractéristique avantageuse de l'invention, ledit emballage extérieur comprend un corps ouvert, de préférence de forme parallélépipédique, dont l'ouverture est refermée par une feuille de scellage appliquée sur la bordure périphérique de l'ouverture.

Avantageusement, ladite ou chaque feuille de scellage est étanche aux liquides et aux bactéries.

L'invention sera bien comprise à la lecture de la description suivante d'exemples de réalisation, en référence aux dessins annexés dans lesquels :
- la figure 1 est une vue en éclaté et à plat d'un emballage intérieur selon un premier mode de réalisation de l'invention comprenant deux parties d'emballage présentant chacune une pluralité de cavités destinées à être refermées par une feuille de scellage adaptée ;
- la figure 2 est une vue de l'emballage intérieur de la figure 1 à l'état plié l'une sur l'autre des deux parties d'emballage de l'emballage intérieur et à l'état logé dans la chambre délimitée par un emballage extérieur dont l'ouverture est destinée à être fermée par une feuille de scellage adaptée;

- la figure 2A est une vue de dessus de l'emballage extérieur et de l'emballage intérieur de la figure 1 ;
- la figure 3 est une vue en éclaté et à plat d'un emballage intérieur selon un deuxième mode de réalisation de l'invention comprenant trois parties d'emballage présentant chacune une pluralité de cavités destinées à être refermées par une feuille de scellage adaptée ;
- la figure 4 est une vue de l'emballage intérieur de la figure 3 à l'état plié l'une sur l'autre de deux parties opposées de l'emballage et à l'état logé dans la chambre délimitée par un emballage extérieur dont l'ouverture est destinée à être fermée par une feuille de scellage adaptée ;
- la figure 5 est une variante de réalisation de l'emballage intérieur de la figure 3 selon laquelle les différentes parties de l'emballage sont disjointes et reliées entre elles par la feuille de scellage.

En référence aux figures et comme rappelé ci-dessus, l'invention concerne un ensemble médical, comprenant un emballage 1 transparent, dit emballage extérieur, délimitant une chambre, et un emballage 2, dit emballage intérieur, logé dans ladite chambre de l'emballage 1 extérieur.

Ledit emballage 1 extérieur comprend un corps ouvert dont l'ouverture 130 est refermée par une feuille de scellage 13 appliquée comme détaillé ci-après sur la bordure 131 périphérique de l'ouverture.

De manière plus générale, l'emballage 1 extérieur est scellé de manière étanche par rapport aux liquides et aux bactéries. La zone de scellage peut être détruite par l'utilisateur pour accéder au contenu dudit emballage extérieur.

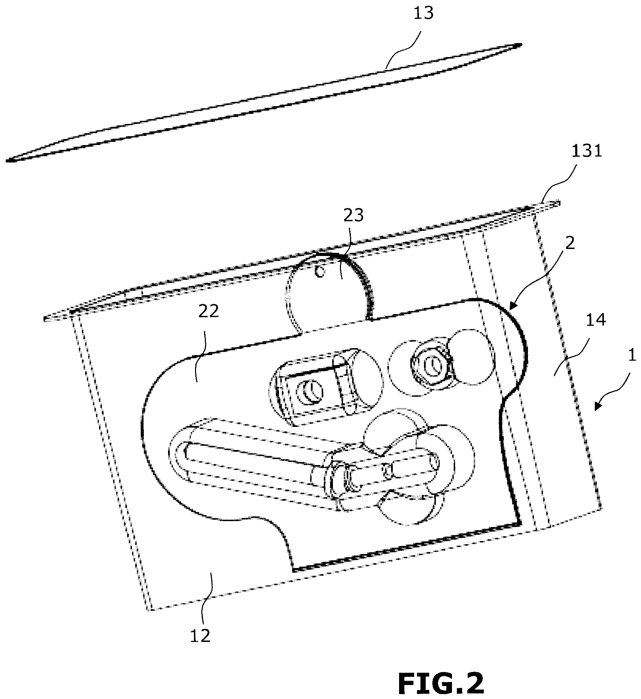

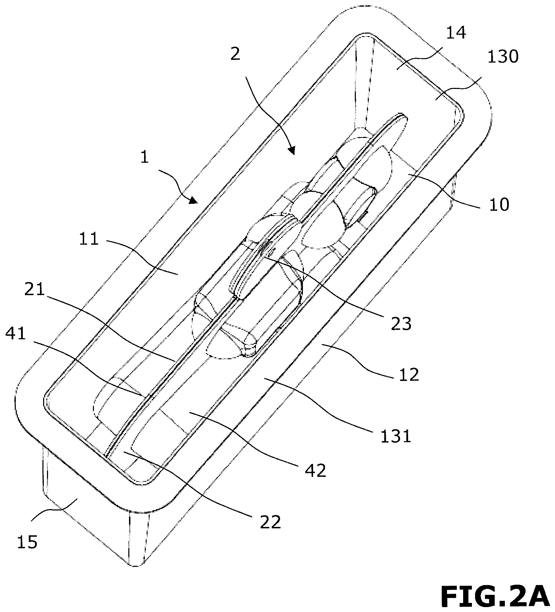

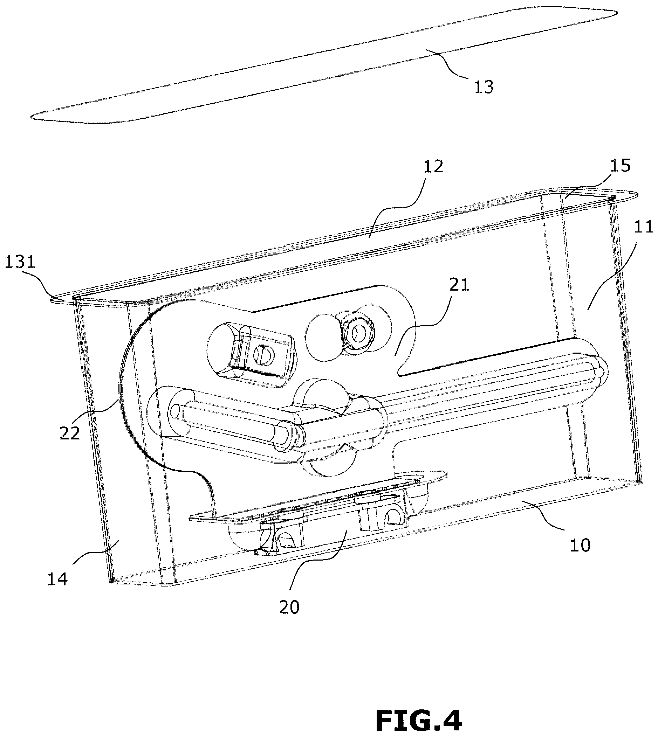

Dans l'exemple illustré aux figures 2, 2A et 4, ledit emballage 1 extérieur se présente ainsi sous la forme d'une boite parallélépipédique dont une face 130 est ouverte et obturée par la feuille de scellage 13. Selon un mode de réalisation, ladite feuille de scellage 13 est au moins partiellement opacifiante par opposition à la transparence de l'emballage.

Ladite feuille de scellage 13 est une feuille thermocollante pelable par exemple en matériau synthétique non-tissé, fabriquée à partir de fibres de polyéthylène, usuellement vendue sous la marque déposée TYVEK.

Ledit emballage 1 extérieur présente ainsi une paroi de fond 10 opposée à l'ouverture 130 de l'emballage 1 extérieur et deux grande parois latérales opposées 11, 12 reliées entre elles par deux autres parois latérales opposées 14, 15 plus petites, appelées parois d'extrémité.

Ledit emballage 2 intérieur comprend au moins une première et une deuxième parties 21, 22 d'emballage qui sont transparentes et qui présentent chacune au moins une cavité 41, 42. Lesdites cavités 41, 42 sont distinctes et séparées, c'est-à-dire qu'elles ne communiquent pas l'une avec l'autre. Chaque cavité contient un objet médical.

Ledit objet peut être par exemple une pièce solide, telle qu'une vis pour chirurgie, ou tout autre type d'objet, en particulier tout autre type d'implant. En outre, ledit objet peut être un liquide ou une poudre. Ledit objet et, de préférence, les différentes parties de l'emballage, sont stérilisés par exemple par rayonnement.

L'ensemble est conçu à des fins de préservation du caractère stérile de chaque objet contenu dans l'emballage 2 intérieur et en vue du déballage de l'objet dans des conditions aseptiques ou proches de l'asepsie.

Dans l'exemple illustré aux figures, chaque partie 21, 22 d'emballage comprend trois cavités 41, 41', 41", respectivement 42, 42', 42", qui logent chacune un objet comme expliqué ci-dessus.

L'emballage 2 intérieur se présente sous la forme d'une plaque pliable dans laquelle sont formées des cavités. Ledit emballage 2 intérieur, de même que l'emballage 1 extérieur, sont de préférence thermoformés. En variante, chaque emballage peut être réalisé par moulage par exemple par injection.

A l'état déplié et à plat de l'emballage 2 intérieur les ouvertures des cavités sont orientées du même côté de ladite plaque formant l'emballage 2 intérieur.

Une feuille de scellage 3 est appliquée sur les deux parties 21, 22 d'emballage de manière à fermer lesdites cavités et à maintenir les objets contenus dedans à l'abri de contaminations extérieures. Ladite feuille de scellage 3 est, comme la feuille de scellage 13, au moins partiellement opacifiante par opposition à la transparence de l'emballage. Avantageusement, ladite feuille de scellage 3 est une feuille thermocollante pelable, par exemple en matériau synthétique non-tissé fabriquée à partir de fibres de polyéthylène, usuellement vendue sous la marque déposée TYVEK.

La feuille de scellage 3 de l'emballage 2 intérieur est appliquée sur lesdites parties d'emballage de l'emballage 2 intérieur sous pression et à chaud, c'est-à-dire par thermoscellage. De même, la feuille de scellage 13 de l'emballage extérieur 1 est aussi appliquée par thermoscellage sur l'ouverture de l'emballage 1 extérieur.

Les deux parties 21, 22 d'emballage sont pliées l'une vers l'autre dans le sens d'une disposition en vis-à-vis des deux parties 31, 32 de la feuille 3 appliquées sur les deux parties d'emballage 21, 22 correspondantes de l'emballage 2 intérieur. Dans l'exemple illustré aux figures, les parties de la feuille de scellage 3 appliquées sur les parties d'emballage de l'emballage intérieur 2 sont réalisées d'une seule pièce les unes avec les autres. Des lignes sécables ou de prédécoupe sont simplement ménagées entre lesdites parties de la feuille 3 de scellage. Selon une variante non illustrée, on peut prévoir que lesdites parties de la feuilles de scellage 3 soient disjointes les unes des autres et qu'ainsi la feuille 3 soit formée de plusieurs pièces.

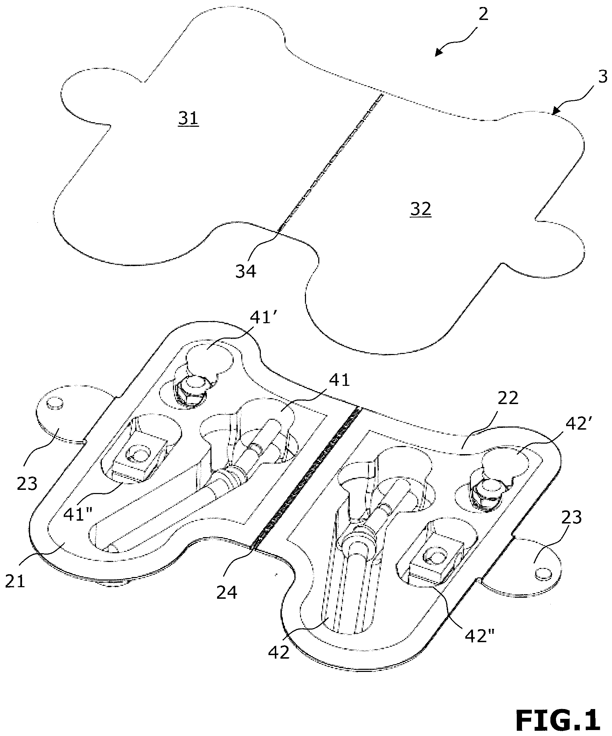

Dans l'exemple illustré aux figures 1, Les première et deuxième parties d'emballage 21, 22 de l'emballage 2 intérieur sont séparées l'une de l'autre par une ligne de pliage 24. De manière similaire, les deux parties 31, 32 de la feuille 3 appliquées sont aussi séparées l'une de l'autre par une ligne de pliage 34.

Ainsi, dans l'exemple illustré aux figures, les deux parties 21, 22 d'emballage de l'emballage 2 intérieur s'étendent l'une 21 en regard de la paroi latérale 11 de l'emballage 1 extérieur et l'autre 22 en regard de l'autre paroi latérale 12 de l'emballage 1 extérieur. Autrement dit, l'une 21 des parties 21, 22 d'emballage est visible d'un côté de l'emballage 1 extérieur et l'autre partie 22 d'emballage est visible de l'autre côté.

Selon une variante non illustrée, on peut prévoir que l'emballage extérieur soit formé de deux coques aptes à délimiter entre elles ladite chambre qui loge l'emballage intérieur.

De manière générale, la chambre délimitée par ledit emballage 1 extérieur est ouvrable par rupture d'une zone de scellage et/ou par séparation de deux parties d'emballage assemblées, telles que deux coques assemblables.

La feuille de scellage 3 n'est pas transparente de sorte que le repli des parties 31, 32 de la feuille 3 sur elles-mêmes permet de positionner cette feuille sensiblement au centre de l'emballage 1 extérieur et d'orienter les parties 21, 22 d'emballage correspondantes de l'emballage 2 intérieur, qui sont transparentes, du côté de la ou des parois de l'emballage 1 extérieur lui aussi transparent.

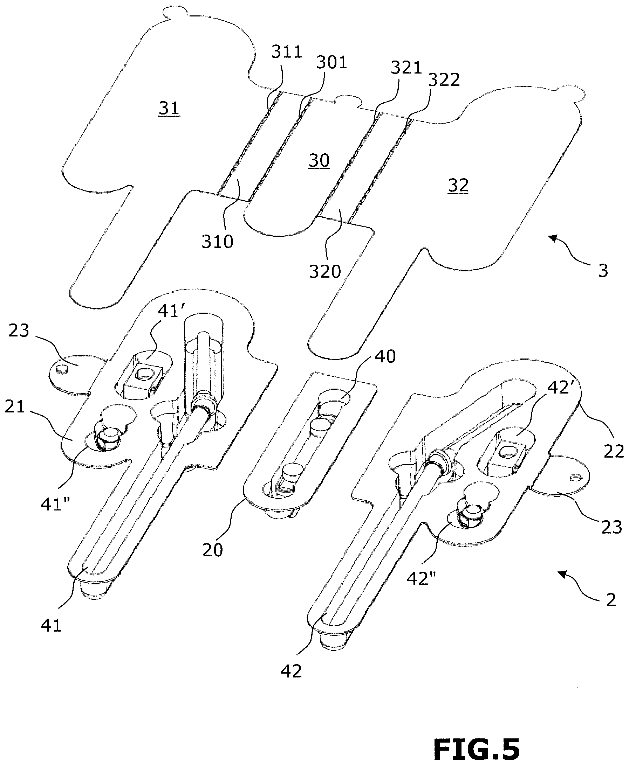

Comme illustré aux figures, l'emballage 2 intérieur comprend des moyens de saisie 23 de l'emballage 2 intérieur accessibles à l'ouverture de l'emballage extérieur 1. Comme rappelé ci-dessus, ladite ouverture 130 de l'emballage extérieur 1 est refermée par la feuille de scellage 13. Ledit emballage comprend aussi des moyens de maintien, de type mâle femelle, des parties d'emballage 21, 22 pliées l'une contre l'autre qui sont désactivables. Dans l'exemple illustré aux figures 1 et 5, ces moyens de maintien sont ménagés sur les moyens de saisie 23 sous forme d'un élément mâle ménagé sur une languette 23 associée à la partie 21 d'emballage et d'un élément femelle ménagé sur une languette 23 associée à la partie 22 d'emballage.

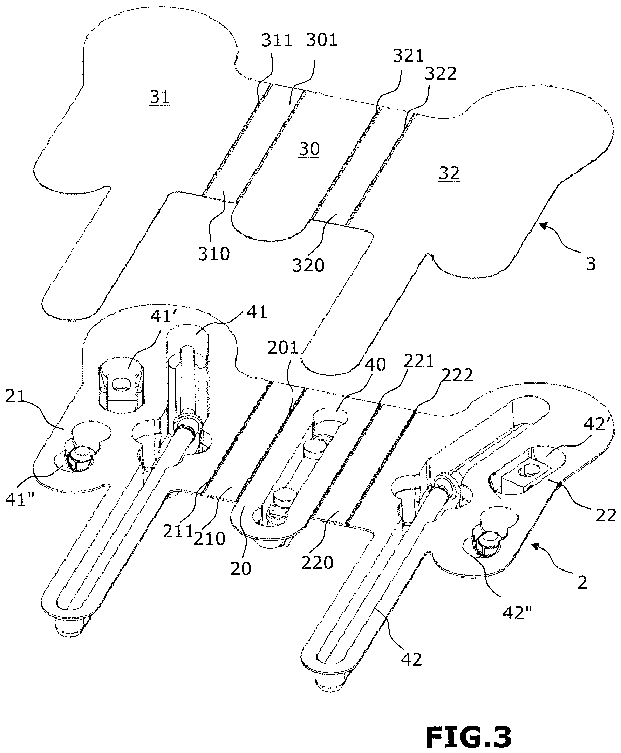

Selon un mode de réalisation illustré aux figures 3 à 5, ledit emballage 2 intérieur comprend une troisième partie d'emballage 20 transparente qui est interposée entre les première et deuxième parties 21, 22 d'emballage. Ladite troisième partie d'emballage 20 comprend une cavité 40 de réception d'objet qui est refermée par une troisième partie 30 de la feuille de scellage 3.

Dans l'exemple illustré aux figures 3 et 4, les première et deuxième parties d'emballage 21, 22 sont séparées chacune de la troisième partie 20 d'emballage par deux lignes de pliage 211, 201, 221, 222 parallèles et écartées l'une de l'autre.

Que ce soit pour le mode de réalisation des figures 1 à 2A ou 3 et 4, la ou chaque ligne de pliage peut être réalisée par une ligne de perforations discontinues permettant non seulement de plier l'emballage autour de cette ligne mais aussi de le déchirer ou de le casser suivant cette ligne.

La feuille de scellage présente aussi des lignes de pliage correspondantes 311, 301 et 321, 322 qui délimitent respectivement entre elles des parties de liaison 310, 320 entre les parties 31, 32 et 30 de la feuille 3 de scellage.

Dans l'exemple illustré aux figures 3 et 4, ladite troisième partie d'emballage 20 de l'emballage 2 intérieur est orientée en regard de la paroi de fond 10 de l'emballage 1 extérieur, tandis que la troisième partie 30 de la feuille 3 de scellage est orientée vers l'ouverture 130 dudit emballage 1 extérieur. Comme pour le mode de réalisation des figures 1 à 2A, les première et deuxième parties 21, 22 de l'emballage intérieur 2 dont les cavités sont refermées par les parties 31, 32 de la feuille 3 de scellage peuvent être pliées l'une vers l'autre dans le sens d'une disposition en vis à vis des parties 31, 32 de la feuille 3. Les parties de liaison 210, 220 de l'emballage 2 intérieur définies entre les lignes de pliages 211, 201 et respectivement 221, 222 sont alors repliées vers la troisième partie 20 de l'emballage 2 intérieur dans le sens d'une disposition des parties de liaison 310, 320 correspondantes de la feuille de scellage en vis à vis de la troisième partie 30 de cette feuille qui recouvre la partie 20 de l'emballage 2 intérieur.

Selon une variante de réalisation illustrée à la figure 5, lesdites parties 20, 21, 22 d'emballage de l'emballage 2 intérieur sont disjointes les unes des autres. La feuille de scellage 3 qui ferme les cavités 40, 41, 41', 41", 42, 42', 42" desdites parties 20, 21, 22 d'emballage relient entre elles lesdites parties 20, 21, 22 de l'emballage 2 intérieur. Autrement dit, les parties disjointes de l'emballage 2 intérieur sont reliées entre elles par une feuille de scellage 3 réalisée d'une seule pièce.

Pour déconditionner l'un ou plusieurs des objets contenus dans l'ensemble et en particulier dans l'emballage 2 intérieur, on peut procéder de la manière suivante.

On entend par personne "sale" ou "contaminée" une personne travaillant dans des conditions non aseptisées, qui est susceptible de contaminer par ses mains les objets qu'elle touche. A l'inverse, on entend par personne "propre" une personne travaillant dans des conditions d'asepsie suffisante.

Après avoir identifié visuellement le ou les objets souhaités à travers l'emballage 1 extérieur et les parties d'emballages 2 intérieur situées en regard de la ou des parois de l'emballage 1 extérieur, une première personne, supposée en zone contaminée, retire la feuille 13 de scellage de l'emballage 1 extérieur pour libérer l'accès à l'emballage 2 intérieur.

Ladite première personne « contaminée » peut ainsi tendre l'emballage 2 intérieur sans le toucher, en tenant l'emballage 1 extérieur. L'emballage 2 intérieur peut alors être saisi par une deuxième personne, supposée en zone propre. Après avoir saisi l'emballage 2 intérieur, de préférence par la zone de saisie 23, ladite personne propre peut alors retirer la ou chaque partie de la feuille 3 de scellage de l'emballage 2 intérieur qui ferme la ou chaque cavité correspondant à l'objet ou à chaque objet souhaité afin de saisir l'objet contenu dans ladite ou chaque cavité. Ladite personne "propre" est par exemple le chirurgien au niveau du bloc opératoire.

On peut aussi prévoir que la personne propre sépare, par exemple par découpage le long des lignes de pliage, la partie de l'emballage qui contient l'objet souhaité de la partie restante de l'emballage intérieur, de manière à n'ouvrir que la cavité renfermant l'objet souhaité.

Ainsi, le ou chaque objet contenu dans une cavité de l'emballage 2 intérieur n'a été ni touché ni lâché au cours de son déconditionnement. En outre, la ou chaque cavité de l'emballage 2 intérieur contenant le ou chaque objet n'est ouverte qu'en zone propre.

## Revendications

1. Ensemble médical, comprenant :
- un emballage (1) transparent, dit emballage extérieur, délimitant une chambre, et
- un emballage (2), dit emballage intérieur, logé dans ladite chambre de l'emballage (1) extérieur,
ledit emballage (2) intérieur comprenant au moins une première et une deuxième parties (21, 22) d'emballage transparentes présentant chacune au moins une cavité (41, 42) pour la réception d'un objet médical, et une feuille de scellage (3), au moins partiellement opacifiante, comprenant deux parties (31, 32) appliquées sur les deux parties (21, 22) d'emballage de manière à fermer lesdites cavités (41, 42),
**caractérisé en ce que** les deux parties (21, 22) d'emballage de l'emballage (2) intérieur sont pliées l'une vers l'autre dans le sens d'une disposition en vis-à-vis des deux parties (31, 32) de ladite feuille de scellage (3) appliquée sur les deux parties d'emballage (21, 22) de l'emballage (2) intérieur.

2. Ensemble selon la revendication 1, **caractérisé en ce que** l'emballage (2) intérieur comprend des moyens de saisie (23) accessibles à l'ouverture de l'emballage extérieur (1).

3. Ensemble selon l'une des revendications précédentes, **caractérisé en ce que** ledit emballage (2) intérieur comprend une troisième partie d'emballage (20) transparente qui s'étend entre les première et deuxième parties (21, 22), ladite troisième partie d'emballage (20) dudit emballage (2) intérieur comprenant au moins une cavité (40) de réception d'objet qui est refermée par une troisième partie (30) de la feuille de scellage (3).

4. Ensemble selon l'une des revendications précédentes, **caractérisé en ce que** les première et deuxième parties d'emballage (21, 22) de l'emballage (2) intérieur sont séparées l'une de l'autre par au moins une ligne de pliage (24).

5. Ensemble selon l'une des revendications précédentes prise en combinaison de la revendication 4, **caractérisé en ce que** les première et deuxième parties d'emballage (21, 22) sont séparées chacune de la troisième partie (20) d'emballage par deux lignes de pliage (211, 201, 221, 222) parallèles et écartées l'une de l'autre.

6. Ensemble selon l'une des revendications précédentes, **caractérisé en ce que** lesdites parties d'emballage (20, 21, 22) de l'emballage (2) intérieur sont réalisées d'un seul tenant.

7. Ensemble selon la revendication 6, **caractérisé en ce que** l'emballage (2) intérieur se présente sous la forme d'une plaque pliable dans laquelle sont formées lesdites cavités (40, 41, 42).

8. Ensemble selon l'une des revendications précédentes, **caractérisé en ce que** lesdites parties (20, 21, 22) d'emballage de l'emballage (2) intérieur sont disjointes les unes des autres,
et **en ce que** ladite feuille de scellage (3) qui ferme les cavités (40, 41, 42) desdites parties d'emballage relient entre elles lesdites parties d'emballage (20, 21, 22) de l'emballage (2) intérieur.

9. Ensemble selon l'une des revendications précédentes, **caractérisé en ce que** la feuille de scellage (3) de l'emballage (2) intérieur est appliquée sur lesdites parties (20, 21, 22) d'emballage de l'emballage (2) intérieur sous pression et à chaud.

10. Ensemble selon l'une des revendications précédentes, **caractérisé en ce que** ledit emballage (1) extérieur comprend un corps ouvert, de préférence de forme parallélépipédique, dont l'ouverture (130) est refermée par une feuille de scellage (13) appliquée sur la bordure périphérique (131) de l'ouverture (130).

11. Ensemble selon l'une des revendications précédentes, **caractérisé en ce que** ladite ou chaque feuille de scellage (3, 13) est étanche aux liquides et aux bactéries.

## Patentansprüche

1. Medizinische Anordnung, umfassend:
- ein transparentes Verpackungselement (1), bezeichnet als äußeres Verpackungselement, das eine Kammer begrenzt, und
- ein Verpackungselement (2), bezeichnet als inneres Verpackungselement, das in der Kammer des äußeren Verpackungselements (1) untergebracht ist,
wobei das innere Verpackungselement (2) mindestens einen ersten Teil und einen zweiten transparenten Verpackungselementteil (21, 22) umfasst, die jeweils mindestens einen Hohlraum (41, 42) zur Aufnahme eines medizinischen Gegenstands umfassen, und eine Abdichtfolie (3), die mindestens teilweise trübend ist, umfassend zwei Teile (31, 32), die auf die zwei Verpackungselementteile (21, 22) derart aufgebracht sind, dass sie die Hohlräume (41, 42) schließen,
**dadurch gekennzeichnet, dass** die zwei Verpackungselementteile (21, 22) des inneren Verpackungselements (2) aufeinander in der Richtung einer Anordnung gegenüber den zwei Teilen (31, 32) der Abdichtfolie (3) gefaltet sind, die auf den zwei Verpackungselementteilen (21, 22) des inneren Verpackungselements (2) aufgebracht sind.

2. Anordnung nach Anspruch 1, **dadurch gekennzeichnet, dass** das innere Verpackungselement (2) Greifmittel (23) umfasst, die bei der Öffnung des äußeren Verpackungselements (1) zugänglich sind.

3. Anordnung nach einem der vorhergehenden Ansprüche, **dadurch gekennzeichnet, dass** das innere Verpackungselement (2) einen dritten transparenten Verpackungselementteil (20) umfasst, der sich zwischen dem ersten und zweiten Teil (21, 22) erstreckt, wobei der dritte Verpackungselementteil (20) des inneren Verpackungselements (2) mindestens einen Hohlraum (40) zur Aufnahme eines Gegenstands umfasst, der durch einen dritten Teil (30) der Abdichtfolie (3) verschlossen ist.

4. Anordnung nach einem der vorhergehenden Ansprüche, **dadurch gekennzeichnet, dass** der erste und zweite Verpackungselementteil (21, 22) des inneren Verpackungselements (2) voneinander durch mindestens eine Faltlinie (24) getrennt sind.

5. Anordnung nach einem der vorhergehenden Ansprüche in Kombination mit Anspruch 4, **dadurch gekennzeichnet, dass** der erste und zweite Verpackungselementteil (21, 22) jeweils vom dritten Verpackungselementteil (20) durch zwei Faltlinien (211, 201, 221, 222) getrennt sind, die parallel und voneinander beabstandet sind.

6. Anordnung nach einem der vorhergehenden Ansprüche, **dadurch gekennzeichnet, dass** die Verpackungselementteile (20, 21, 22) des inneren Verpackungselements (2) aus einem Stück hergestellt sind.

7. Anordnung nach Anspruch 6, **dadurch gekennzeichnet, dass** das innere Verpackungselement (2) die Form einer faltbaren Platte aufweist, in der die Hohlräume (40, 41, 42) gebildet sind.

8. Anordnung nach einem der vorhergehenden Ansprüche, **dadurch gekennzeichnet, dass** die Verpackungselementteile (20, 21, 22) des inneren Verpackungselements (2) voneinander getrennt sind,
und dadurch, dass die Abdichtfolie (3), die die Hohlräume (40, 41, 42) der Verpackungselementteile schließt, die Verpackungselementteile (20, 21, 22) des inneren Verpackungsteils (2) untereinander verbindet.

9. Anordnung nach einem der vorhergehenden Ansprüche, **dadurch gekennzeichnet, dass** die Abdichtfolie (3) des inneren Verpackungselements (2) auf die Verpackungselementteile (20, 21, 22) des inneren Verpackungselements (2) unter Druck und heiß aufgebracht wird.

10. Anordnung nach einem der vorhergehenden Ansprüche, **dadurch gekennzeichnet, dass** das äußere Verpackungselement (1) einen offenen Körper umfasst, vorzugsweise in parallelepipeder Form, dessen Öffnung (130) durch eine Abdichtfolie (13) verschlossen ist, die auf den peripheren Rand (131) der Öffnung (130) aufgebracht ist.

11. Anordnung nach einem der vorhergehenden Ansprüche, **dadurch gekennzeichnet, dass** die oder jede Abdichtfolie (3, 13) gegen Flüssigkeiten und Bakterien dicht ist.

## Claims

1. A medical assembly, comprising:
- a transparent package (1), called outer package, delimiting a chamber, and
- a package (2), called inner package, housed in said chamber of the outer package (1),
said inner package (2) comprising at least a first and a second transparent package part (21, 22) each having at least one cavity (41, 42) for receiving a medical object, and a sealing sheet (3), at least partially opacifying, comprising two parts (31, 32) that are applied on the two package parts (21, 22) so as to close said cavities (41, 42),
**characterized in that** the two package parts (21, 22) of the inner package (2) are folded toward one another in the direction of a facing arrangement of the two parts (31, 32) of said sealing sheet (3) applied on the two package parts (21, 22) of the inner package (2).

2. The assembly according to claim 1, **characterized in that** the inner package (2) comprises gripping means (23) that are accessible at the opening of the outer package (1).

3. The assembly according to one of the preceding claims, **characterized in that** said inner package (2) comprises a third transparent package part (20) that extends between the first and second parts (21, 22), said third package part (20) of said inner package (2) comprising at least one cavity (40) for receiving an object that is closed by a third part (30) of the sealing sheet (3).

4. The assembly according to one of the preceding claims, **characterized in that** the first and second package parts (21, 22) of the inner package (2) are separated from one another by at least one folding line (24).

5. The assembly according to one of the preceding claims taken in combination with claim 4, **characterized in that** the first and second package parts (21, 22) are each separated from the third package part (20) by two parallel folding lines (211, 201, 221, 222) that are separated from one another.

6. The assembly according to one of the preceding claims, **characterized in that** said package parts (20, 21, 22) of the inner package (2) are made in a single piece.

7. The assembly according to claim 6, **characterized in that** the inner package (2) assumes the form of a foldable plate in which the cavities (40, 41, 42) are formed.

8. The assembly according to one of the preceding claims, **characterized in that** said package parts (20, 21, 22) of the inner package (2) are separated from one another,
and **in that** said sealing sheet (3) that closes the cavities (40, 41, 42) of said package parts connects said package parts (20, 21, 22) of the inner package (2) to one another.

9. The assembly according to one of the preceding claims, **characterized in that** the sealing sheet (3) of the inner package (2) is applied on said package parts (20, 21, 22) of the inner package (2) under pressure and hot.

10. The assembly according to one of the preceding claims, **characterized in that** said outer package (1) comprises an open body, preferably of parallelepipedal shape, the opening (130) of which is closed by a sealing sheet (13) applied on the peripheral border (131) of the opening (130).

11. The assembly according to one of the preceding claims, **characterized in that** said or each sealing sheet (3, 13) is sealed against liquids and bacteria.
